**Europäisches Patentamt**

**European Patent Office**

**Office européen des brevets**

(11) Veröffentlichungsnummer: **0 240 787**
**A2**

(12) **EUROPÄISCHE PATENTANMELDUNG**

(21) Anmeldenummer: **87103912.9**

(22) Anmeldetag: **17.03.87**

(51) Int. Cl.⁴: **A61M 5/32**

(30) Priorität: **09.04.86 US 849722**

(43) Veröffentlichungstag der Anmeldung:
**14.10.87 Patentblatt 87/42**

(84) Benannte Vertragsstaaten:
**BE CH DE ES FR GB IT LI NL SE**

(71) Anmelder: **Pharma-Gummi Wimmer West GmbH**
**Stolberger Strasse 21-41**
**D-5180 Eschweiler(DE)**

(72) Erfinder: **Lowe, Allen D.**
**R.D. 2 Box 623**
**Montgomery Pennsylvania 17752(US)**
Erfinder: **Brown, Homer J., Jr.**
**526 Marks Road**
**Oreland Pennsylvania 19075(US)**
Erfinder: **Harmon, Arlington R., Jr.**
**27 Spring Valley Road**
**Frazer Pennsylvania 19355(US)**

(74) Vertreter: **Schmitt, Hans, Dipl.-Ing. et al**
**Patentanwälte Dipl.-Ing H. Schmitt Dipl.-Ing.**
**W. Maucher Dreikönigstrasse 13**
**D-7800 Freiburg(DE)**

(54) Nadelschutzhülle für Parenteral-Nadeln und Verfahren zum Herstellen von Nadelschutzhüllen.

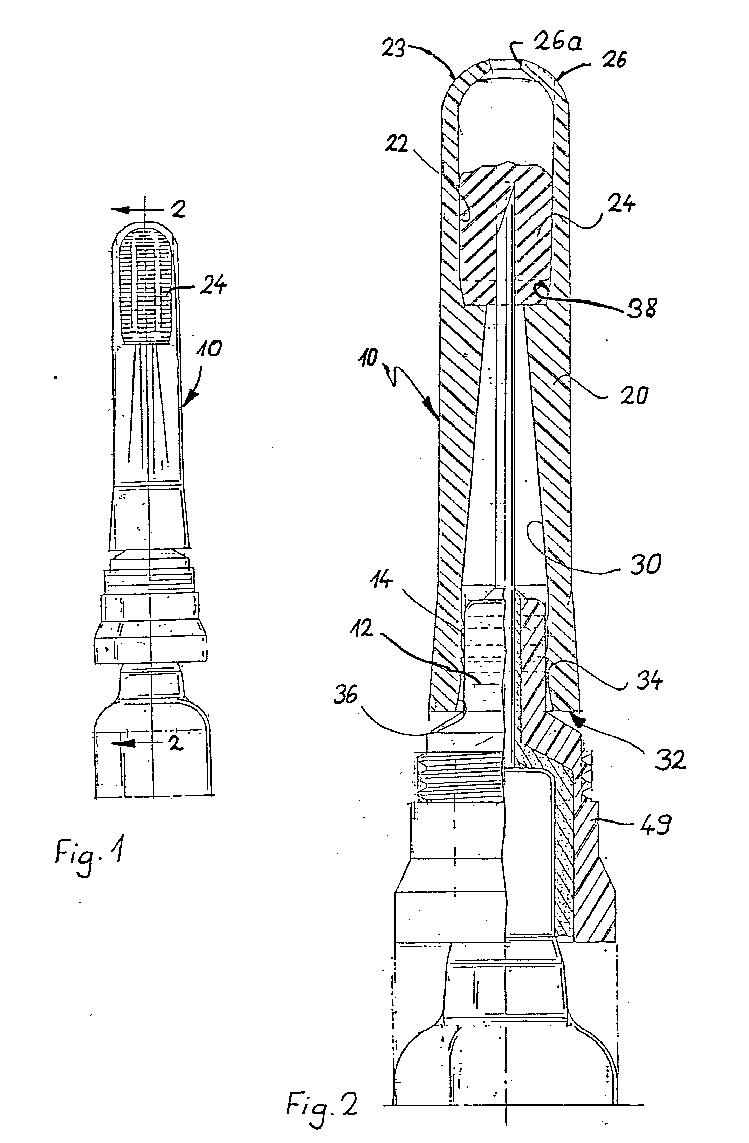

(57) Eine Nadelschutzhülle zum Schutz von Parenteral-Nadeln mit einem länglichen, rohrförmigen Gehäuse (20) und konisch sich verengender, zentraler Bohrung (30) weist an einem Ende ein Befestigungselement zur abnehmbaren Befestigung auf dem Sockel (49) einer Spritzenanordnung auf. Am befestigungsabgewandten Ende des Gehäuses (20) befindet sich ein mit der Bohrung (30) in Verbindung stehender Aufnahmeraum (22) zur Aufnahme eines aus elastischem, durchstechbaren Material bestehenden Einsatzteiles (24) das in engem Wandabschluß in dem Aufnahmeraum (22) gehalten ist. Neben einem sauberen Eindringen der Nadel in das Einsatzteil wird durch diese Konstruktion vor allem ein preiswertes und sicheres Herstellungs-und Montageverfahren ermöglicht (Fig.2).

EP 0 240 787 A2

Fig. 2

## Nadelschutzhülle für Parenteral-Nadeln und Verfahren zum Herstellen von Nadelschutzhüllen

Die Erfindung betrifft eine Nadelschutzhülle mit einem länglichen, rohrförmigen Gehäuse mit zentraler Bohrung und einem an einem Ende sich befindenden Befestigungselement, womit die Schutzhülle auf dem Sockel einer Spritzenanorndung abnehmbar befestigt ist.

Nadelschutzhüllen solcher Art sind nicht neu. Z. B. zeigt das US-Patent Nr. 2 831 483 eine Kunststoffhülle mit einer im spitzen Ende der Haube angebrachten Masse elastischen Materials. Das US-Patent Nr. 3 370 588 zeigt einen Nadelschutz, der einen Spielraum zwischen der Nadelspitze und der Schutzhaubenspitze vorsieht. Das US-Patent Nr. 4 249 530 zeigt eine ähnliche Anordnung. Auch das US-Patent Nr. 4 402 682 zeigt einen Kunststoff/Gummi-Nadelschutz. Andere, den Stand der Technik bestimmende Patente seien im folgenden tabellarisch aufgeführt :

| Erfinder | Patent Nr. | Erteilungsdatum |
|----------|------------|-----------------|
| MacGregor | 1,331,271 | 17. Februar 1920 |
| Jaros | 1,589,969 | 22. Juni 1926 |
| Saffir | 2,512,568 | 20. Juni 1950 |
| Saffir | 2,512,569 | 20. Juni 1950 |
| Hamilton | 2,933,087 | 19. April 1960 |
| Jacob | 3,186,408 | 01. Juni 1965 |
| Bradley et al | 3,390,678 | 02. Juli 1968 |
| Kitay | 3,430,627 | 04. März 1969 |

Diese vorbekannten Nadelschutzhüllen haben jedoch noch einige Nachteile. Es erweist sich bei ihnen insbesondere als zeitaufwendig und schwierig, die vorbeschriebenen elastischen Einsätze in das Nadelschutzgehäuse einzumontieren. Zudem eignen sich diese bekannten Konstruktionen nicht ohne weiteres für hochgeschwindigkeits-automatisierte Montagemaschinen und -techniken. Zudem ist der elastische Einsatz in diesen Nadelschutzhüllen nicht gegen axiale Verschiebungen gesichert. Aus diesem Grunde besteht bei den vorbekannten Nadelschutzhüllen die Gefahr, daß die Nadelspitze nicht so wie vorgesehen in das Einsatzteil eindringt und dort nicht sauber sitzt.

Des weiteren bewirkt der schräge Anschliff der Injektionskanüle (Nadelspitze) beim Eindringen in elastische Materialien ein seitliches Ausweichen, das zu schiefer Positionierung der Nadelschutzhülle oder gar zum seitlichen Durchstechen führen kann.

Unter diesen Voraussetzungen kann der elastische Einsatz die Nadelspitze nicht mehr gleichmäßig und im erforderlichen Maße umschließen, so daß der keimfreie Abschluß der Nadel gefährdet sein kann.

Es besteht deshalb insbesondere die Aufgabe, eine Nadelschutzhülle der eingangs erwähnten Art zu schaffen, die die genannten Nachteile vermeidet, insbesondere die Sicherheit eines keimfreien Abschlusses der Nadelspitze erhöht und eine leichte und preiswerte Herstellung ermöglicht.

Erfindungsgemäß wird diese Aufgabe insbesondere dadurch gelöst, daß sich die Bohrung konisch verengt, und daß am befestigungsabgewandten Ende des rohrförmigen Gehäuses ein mit der Bohrung in Verbindung stehender Raum zur Aufnahme eines aus elastischem Material bestehenden Einsatzteiles ausgebildet ist, das in engem Wandabschluß in dem Raum gehalten ist.

Wie weiter unten noch dargestellt wird, eignet sich diese Konstruktion für eine einfache und automatisierungsfähige preiswerte Herstellung. Der enge Wandabschluß des elastischen Einsatzteiles verhindert ein axiales Spiel und sorgt somit für ein sauberes Eindringen und einen sauberen Sitz der Nadel.

Die Erfindung betrifft auch ein Verfahren zur Herstellung einer Nadelschutzhülle. Dieses Verfahren ist insbesondere dadurch gekennzeichnet, daß zunächst ein längliches, rohrförmiges Gehäuse ausgeformt wird, welches eine spitz zulaufende Zentralbohrung und an einem axialen Ende des Gehäuses einen im wesentlichen zylinderischen Aufnahmeraum aufweist, und daß daraufhin ein im wesentlichen zylinderisches Einsatzteil in den Aufnahmeraum des Gehäuses eingesetzt wird und darauffolgend das Wandende des Aufnahmeraumes verformt wird, um das Einsatzteil darin einzukapseln.

0 240 787

Die Einfachheit der einzelnen Verfahrensschritte erlaubt eine sehr schnelle automatisierte Herstellung der erfindungsgemäßen Nadelschutzhülle bei gleichbleibend sicherer und guter Verarbeitung. Nachdem das Einsatzteil in den im wesentlichen zylinderischen Raum eingesetzt worden ist, kann die äußere abschließende Kante des Aufnahmeraumes z. B. unter Anwendung von Hitze und Druck in die lichte Öffnung hineingebogen werden. Insbesondere dann, wenn das innere Ende des Aufnahmeraumes leicht zugespitzt oder konisch verengt ist, kann eine dichte Versiegelung zwischen dem Einsatzteil und der äußeren Wand erreicht werden.

Nachstehend ist die Erfindung mit den ihr als wesentlich zugehörigen Einzelheiten anhand von erfindungsgemäßen Ausführungsbeispielen und mit Hilfe der Zeichnungen noch näher beschrieben.

Es zeigen :

Fig. 1 eine schematisch aufrechte Teil-Seiten-ansicht einer erfindungsgemäßen Nadelschutzhülle, die auf den Sockel einer typischen Spritzenanordnung aufgesetzt ist,

Fig. 2 eine vergrößerte schematische Schnittansicht entsprechend der Linien 2-2 von Fig. 1, die die inneren Einzelheiten der Nadelschutzhülle zeigt,

Fig. 3 ein vergrößerte Schnittansicht in schematischer Darstellung, die die Montage der Einzelteile der Nadelschutzhülle zeigt,

Fig. 4 eine erfindungsgemäße Nadelschutzhülle, die auf einen anderen Sockeltyp einer Injektionsnadel aufgesetzt ist,

Fig. 5 eine vergrößerte schematische Schnittansicht entsprechend den Linien 5-5 von Fig. 4,

Fig. 6 eine Draufsicht auf eine Nadelschutzhülle nach Fig. 4 und

Fig. 7 eine vergrößerte schematische Schnittansicht des sockelseitigen Endes der Nadelschutzhülle nach Fig. 4.

Fig. 1 bis 3 zeigen eine erfindungsgemäße, im ganzen mit 10 bezeichnete Nadelschutzhülle. Sie ist zur Anbringung auf den Sockel 12 einer Spritzenanordnung vorgesehen, der eine ziemlich glatte äußere zylinderische Oberfläche 14 aufweist.

Die Nadelschutzhülle 10 ist vorzugsweise aus starrem Kunststoffmaterial, z. B. aus Polypropylen, hergestellt und weist ein längliches, rohrförmiges Gehäuse 20 mit einem Aufnahmeraum 22 auf, der an einem distalaxialen Ende 23 dieses Gehäuses 20 ausgeformt ist und einen Gummieinsatz 24 enthält. Wie dargestellt, ist die Wandung des Gehäuses an seinem äußeren, sockelabgewandten Ende 26 abgerundet, um eine Umhüllung für den Einsatz, wie unten im einzelnen erklärt, zu bilden. Der Aufnahmeraum 22 steht in Verbindung mit einer länglichen zentralen Bohrung 30, die wie dargestellt, einen konisch zusammenlaufenden Querschnitt aufweist, der im vorliegenden Ausführungsbeispiel in Richtung des Aufnahmeraumes 22 des Gehäuses 20 radial nach innen zusammenläuft. Das offene spritzenseitige Ende 32 des Gehäuses 20, das über den Nadelsockel 12 paßt, hat eine Reihe von konzentrischen,axial beabstandeten Rippen 34 sowie einen nach außen auseinanderlaufenden Führungsbereich 36, der sich bis zum lichten offenen Ende der Sockelseite der Nadelschutzhülle 10 erstreckt, um das Aufsetzen auf die Spritzenanordnung zu erleichtern. Der Aufnahmeraum 22 für den Gummieinsatz 24 weist einen im wesentlichen gleichmäßigen Durchmesser D 2 über seine Länge auf. An seinem inneren,spritzenzugewandten axialen Ende 39 ist er jedoch radial nach innen leicht verengt, um eine feste und effektive Versiegelung nach der Montage des Einsatzes 24 zu erreichen.

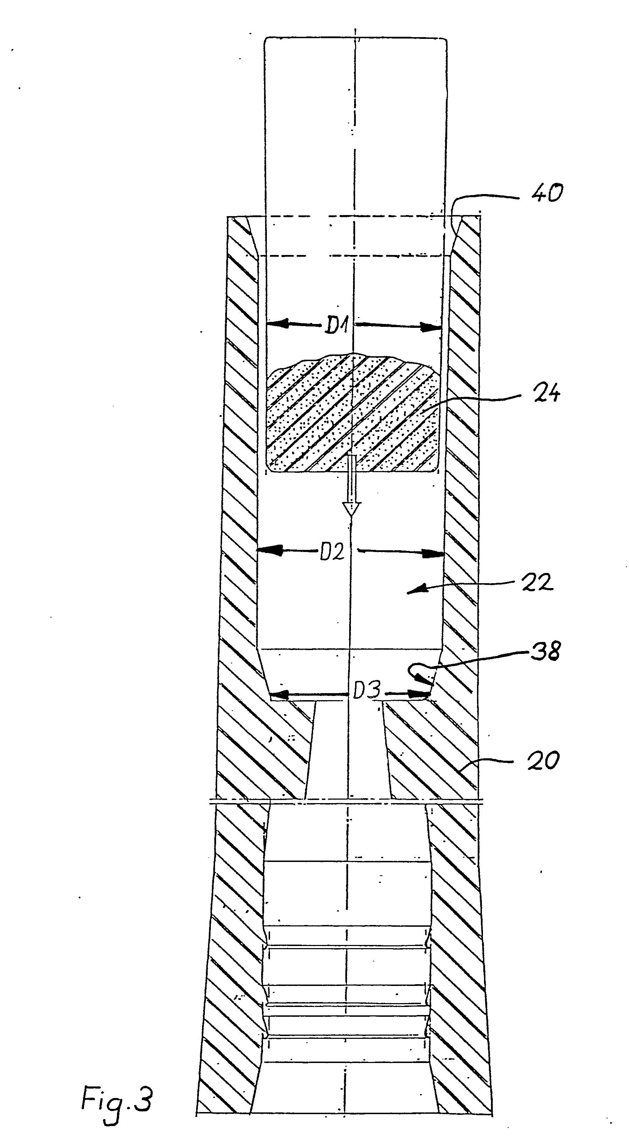

Das distale Ende 23 des Gehäuses 20 ist im wesentlichen zylinderisch und umgrenzt eine zunächst offene Kammer, in die der Gummieinsatz 24 verformungslos eingesetzt werden kann. Dabei vereinfacht die leichte Schräge 40 der Endkante der Gehäusewandung das Einführen dieses Gummieinsatzes 24, der, wie dargestellt, eine im wesentlichen zylinderische Formgebung sowie einen gleichbleibenden Durchmesser über seine gesamte Länge aufweist. Der Durchmesser D 1 des Einsatzes 24 ist etwas kleiner als der Hauptdurchmesser D 2 des Aufnahmeraumes 22 und größer als der kleinste Durchmesser D 3 des konisch verengten Bodenbereiches 38 des Aufnahmeraumes 22, an den sich die Zentralbohrung 30 des Gehäuses 20 anschließt. Nachdem der Einsatz 24 eingeführt worden ist, wird das distale Ende 23 des Gehäuses 20 durch Anwendung von Hitze und Druck in die Form gebracht, wie sie in Fig. 2 gezeigt ist, um hierdurch den Einsatz 24 einzukapseln und festzulegen, indem die spritzenabgewandte lichte zylinderische Öffnung der Nadelschutzhülle (vergl. Fig. 3) radial nach innen verformt und in die in Fig. 2 dargestellte Endform gebracht wird. Vor dieser Verformung wird der Einsatz 24 vorkomprimiert, indem ein Kompressionsstift benutzt wird, der entlang der zentralen Achse des Einsatzes 24 durch die Öffnung 26a wirkt. Während dieser Kompressionsphase wird die äußere abschließende Wandung des Gehäuses, wie beschrieben, verformt. Die Öffnung 26a wird im distalen Ende 23 der Öffnung eingeformt, sobald im folgenden Schritt der Kompressionsstift aus der Öffnung herausgezogen ist. Dieses Verfahren hat den Vorteil, daß es an Hochgeschwindigkeitsautomaten zur Produktion von Nadelschutzhüllen mit gleichbleibend hermetischer Abdichtung angepaßt werden kann .

3

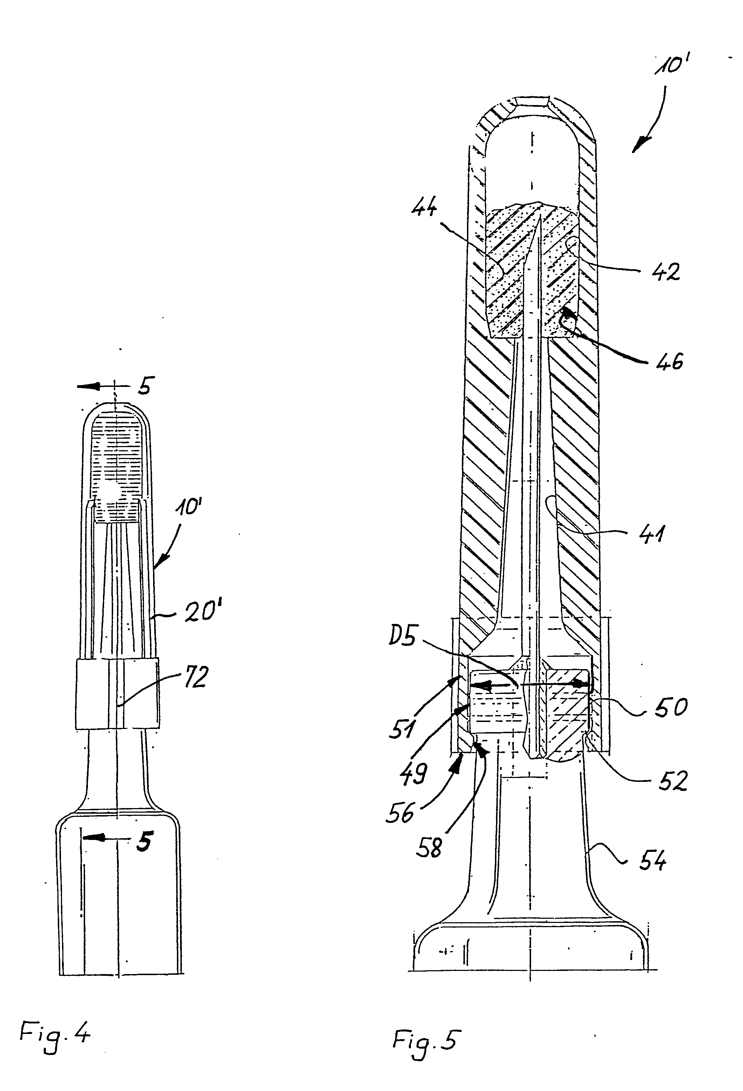

Fig. 4 bis 7 zeigen eine abgewandelte Art von erfindungsgemäßen Nadelschutzhüllen 10'. Die Grundausgestaltung des Gehäuses 20' und des Einsatzes 44 ist im allgemeinen ähnlich derjenigen, die in Verbindung mit der prinzipiellen Ausgestaltung bereits beschrieben worden ist. So enthält das Gehäuse 20' eine längliche, zugespitzte zentrale Bohrung 41 und so hat auch der Aufnahmeraum 42 für den Einsatz 44 einen schmalen, kegelstumpfförmigen Boden -bereich 46, um einen festen und dichten Sitz zu gewährleisten, wenn der Gummieinsatz 44 auf die wesentlichen gleiche Weise wie vorstehend beschrieben, montiert wird. Im vorliegenden Beispiel ist die Nadelschutzhülle 10' zum Aufsetzen auf einen Nadelsockel 49 in einer Ausgestaltung ausgelegt, wie sie in Fig. 5 dargestellt ist. Zu beachten ist, daß der Nadelsockel 49 ein vergrößertes Kopfteil 50 aufweist, welches eine sich kreisförmige erstreckenden Schulter 52 miteinem Halsteil 54 des Spritzenrumpfes umgrenzt. Im gezeigten Beispiel weist das bodenseitige Ende 56 des Gehäuses der Nadelschutzhülle 10' eine radial einwärts gerichtete, an sein offenes abschließendes Ende angrenzende Rippe 58 auf, die einen inneren Durchmesser D 4 hat, der kleiner ist als der Maximaldurchmesser D 5 des Sockels 49. Die Rippe 58 weist zur Spritze hin eine leichte, den Durchmesser vergrößernde Abschrägung 62 auf, um das Aufschieben des Randwulstes über den Nadelsockel 49 zu erleichtern. Die innere Wand 64 des Aufsetzbereiches 65 der Nadelschutzhülle 10' hat im vorliegenden Beispiel eine Reihe von konzentrisch radial einwärts gerichteten Dichtwölbungen 66 mit einem im Vergleich zum Durchmesser D 5 des Sockels 49 etwas kleineren Durchmesser D 6, um den Sockel 49 eng zu umgreifen und einen dichtenden Sitz in der in Fig. 5 dargestellten Art zu gewährleisten.

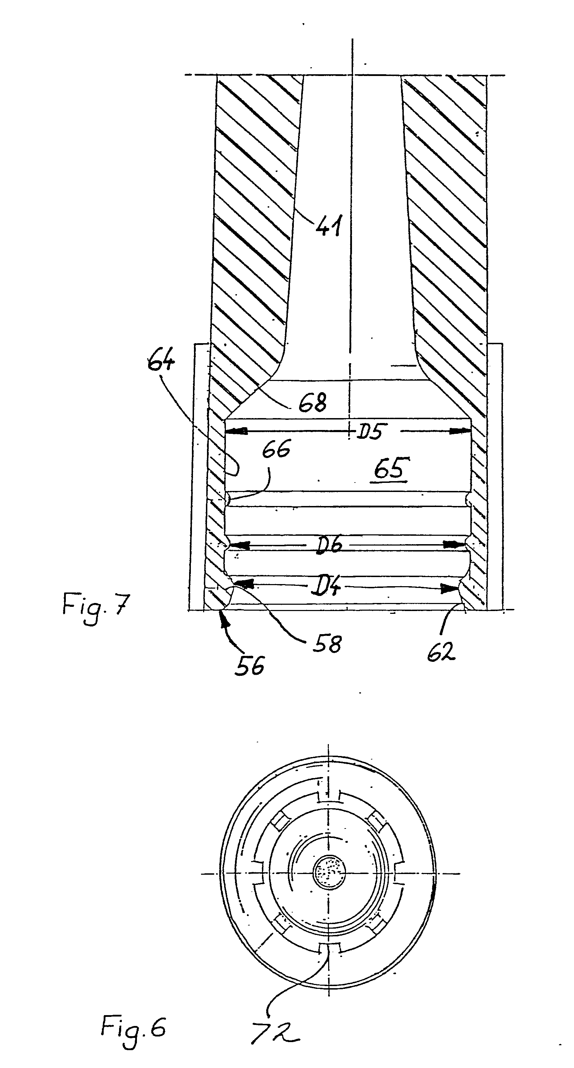

Die inneren Wände des Aufsetzbereiches 65 enden in einer leicht gekrümmten Verbindungswand 68, die in die spitz zulaufende zentrale Bohrung 41 übergeht, durch welche die Nadel beim Aufsetzen der Nadelschutzhülle 10' geführt wird (Fig. 7).

Wie in Fig. 5 bis 7 dargestellt, hat der Aufsetzbereich 51 des Gehäuses 20' eine Reihe von axial sich erstreckenden, auf dem Umfang gleichmäßig beabstandete Nuten 72, die dem Aufsetzbereich 51 einen gewissen Grad an Flexibilität verleihen. Durch dieses Konstruktionsmerkmal wird ein festes Aufsetzen auf den Aufsetzbereich 51 sogar während Autoclav-Verfahren gewährleistet. Diese Aussparungen oder Keilnuten gleichen auch praktisch jegliche herstellungsbedingtenToleranzen zwischen Nadel-Sockel 49 und den Dichtrippen 66 aus.

Zur Formung des Distalendes 23 des Gehäuses 20 kann ein Einheitsformwerkzeug benutzt werden, welches eine kuppelförmige Ausnehmung aufweist, die zur abschließend gerundeten Form des distalen Endes 23 des Gehäuses 20 paßt sowie ein axial aus der Kuppel zentral herausstehender Stift. Die nach Fertigstellung der Nadelschutzhülle sich anschließende Montage auf eine Spritzenanordnung wird durch die - eine Führung der Nadel bewirkende -konische Verengung der Zentralbohrung 30 erleichtert. Im Aufsetzbereich 51 des Gehäuses 20' (Fig. 5 bis 7) sind eine Reihe von vorzugsweise axialen Nuten 72 od. dgl. Aussparungen vorgesehen. Diese dienen der Vergrößerung der Flexibilität dieses Aufsetzbereiches 51. Dabei sind diese Nuten 72 so dimensioniert, daß das Aufsetzen selbst während der Ausdehnungsphase im Autoclav-Verfahren gewährleistet ist.

Alle in den Ansprüchen und in der Beschreibung genannten Merkmale können einzeln oder im Zusammenhang erfindungswesentlich sein.

## Ansprüche

1. Nadelschutzhülle (10) mit einem länglichen, rohrförmigen Gehäuse (20) mit zentraler Bohrung (30, 41) und einem an einem Ende sich befindenden Befestigungselement, womit die Schutzhülle (10) auf dem Sockel (49) einer Spritzenanordnung abnehmbar befestigt ist, **dadurch gekennzeichnet,** daß am befestigungsabgewandten Ende des rohrförmigen Gehäuses (20) ein mit der Bohrung (30, 41) in Verbindung stehender Aufnahmeraum (22, 42) zur Aufnahme eines aus elastischem, durchstechbaren Material bestehenden Einsatzteiles (24, 44) ausgebildet ist, das in engem Wandabschluß in dem Aufnahmeraum (22, 42) gehalten ist.

2. Nadelschutzhülle nach Anspruch 1, dadurch gekennzeichnet, daß der Aufnahmeraum (22, 42) eine im wesentlichen zylinderische Form mit einem Durchmesser (D 2) aufweist, der etwas größer ist als der Durchmesser (D 1) des Einsatzteiles (24, 44) und einen konisch sich verengenden Bodenbereich (38) aufweist, der einen Abschnitt von gegenüber dem Einsatzteil (24, 44) kleinerem Durchmesser (D 3) hat, um eine feste, dichtende Verbindung mit diesem zu gewährleisten.

3. Nadelschutzhülle nach Anspruch 1 oder 2, dadurch gekennzeichnet, daß sie im Aufsetzbereich (51) des Gehäuses (20') eine Reihe von vorzugsweise axiale Nuten 72 od. dgl. Aussparungen aufweist, die zur Vergrößerung der Flexibilität des Aufsetzbereiches (51) vorzugsweise so dimensioniert sind, daß das Aufsetzen selbst während der Ausdehnungsphase in Autoclav-Verfahren gewährleistet ist.

4. Verfahren zur Herstellung einer Nadelschutzhülle, dadurch gekennzeichnet, daß zunächst ein längliches rohrförmiges Gehäuse (20) ausgeformt wird, welches eine spitz zulaufende Zentralbohrung (30, 41) und an einem axialen Ende (23) des Gehäuses (20) einen im wesentlichen zylindrischen Aufnahmeraum (22, 42) aufweist, und daß daraufhin ein im wesentlichen zylindrischer Einsatz (24, 44) in den Aufnahmeraum (22, 42) eingesetzt wird und darauffolgend das offene Wandende des Raumes (22, 42) zur Einkapselung des Einsatzes (24, 44) verformt wird.

5. Verfahren nach Anspruch 4, dadurch gekennzeichnet, daß der Einsatz (24, 44) vor der Verformung des Gehäuses (20) vorkomprimiert wird.

Fig. 1

Fig. 2

Fig.3

Fig. 4

Fig. 5

0 240 787

Fig. 7

Fig. 6